Europäisches Patentamt

(19) **European Patent Office**

**Office européen des brevets**

(11) Numéro de publication: **0 065 905**
**B1**

(12) # FASCICULE DE BREVET EUROPEEN

(45) Date de publication du fascicule du brevet:
**10.10.84**

(51) Int. Cl.³: **A 61 K 39/00,** A 61 K 39/12,
A 61 K 39/20, A 61 K 9/14

(21) Numéro de dépôt: **82400857.7**

(22) Date de dépôt: **10.05.82**

(54) **Perfectionnements apportés aux agents de stabilisation de virus vivants pour la préparation de vaccins, et vaccins stabilisés contenant lesdits agents de stabilisation.**

(30) Priorité: **13.05.81 FR 8109490**

(43) Date de publication de la demande:
**01.12.82 Bulletin 82/48**

(45) Mention de la délivrance du brevet:
**10.10.84 Bulletin 84/41**

(84) Etats contractants désignés:
**BE CH DE GB IT LI NL**

(56) Documents cités:
**EP - A - 0 028 563**
**FR - A - 2 076 787**
**FR - A - 2 215 946**
**FR - A - 2 424 031**
**US - A - 3 873 421**

(73) Titulaire: **INSTITUT PASTEUR, 25-28, rue du Docteur Roux, F-75724 Paris Cedex 15 (FR)**

(72) Inventeur: **Barme, Michel, 11, rue de l'Armorique, F-75015 Paris (FR)**

(74) Mandataire: **Ores, Irène et al, CABINET ORES 6, Avenue de Messine, F-75008 Paris (FR)**

BUNDESDRUCKEREI BERLIN

## Description

La présente invention est relative à un nouvel agent de stabilisation de virus vivants en vue de l'utilisation de ces derniers en tant que vaccins et aux vaccins stabilisés ainsi obtenus.

On utilise depuis longtemps pour protéger l'homme et l'animal contre les maladies virales, la vaccination par des antigènes inactivés ou des souches virales atténuées vivantes. Dans les deux cas, il est impératif que l'intégrité des antigènes viraux soit conservée dans les vaccins pour que l'action vaccinante de ces derniers soit efficace après préparation, stockage et transport des vaccins sur les lieux de la vaccination. Or, les vaccins atténués vivants sont particulièrement fragiles et leur action vaccinante est rapidement détruite lorsqu'ils sont exposés à des températures supérieures à +4°C et même parfois moins. Une telle instabilité des vaccins est inacceptable, notamment lorsque ces vaccins sont utilisés, comme le vaccin anti-amaril (vaccin contre la fièvre jaune), à grande échelle dans des pays tropicaux où ils peuvent accidentellement être exposés aux températures élevées ambiantes qui règnent dans ces régions, avant d'être administrés, attendu que le maintien de la stabilité du titre des vaccins vivants est le facteur primordial pour assurer une immunisation efficace des populations vaccinées, tout particulièrement dans les régions à climat chaud, et notamment dans les cas où la chaîne du froid depuis le laboratoire de fabrication jusqu'aux lieux d'utilisation peut s'avérer insuffisante ou être interrompue. Il est donc nécessaire de pouvoir disposer d'un vaccin stable et thermorésistant, capable de résister à la détérioration lorsqu'il est stocké pendant de longues durées et est exposé accidentellement à des températures élevées. Pour conférer aux vaccins vivants la thermostabilité indispensable au maintien de l'intégralité de leur pouvoir vaccinant — ou infectant —, il a été proposé de préparer ces vaccins, notamment les vaccins contre la rougeole, la rubéole, les oreillons, la fièvre jaune, sous forme lyophilisée. Toutefois, même sous cette forme stabilisée, ces vaccins vivants lyophilisés sont encore très fragiles au point qu'ils doivent être conservés à −30°C et sont expédiés dans de l'anhydride carbonique solide (plus connu sous la Marque »CARBOGLACE« ®) à −80°C. Toutefois, même en se soumettant à ces contraintes de stockage et d'expédition extrêmement drastiques, on ne peut pas toujours éviter que les vaccins soient exposés, au cours de leur stockage et de leur expédition, à des températures élevées qui provoquent la perte du pouvoir infectant de ces vaccins et, donc, leur perte d'activité, en sorte que les populations vaccinées ne sont, en fait, nullement protégées par une vaccination par un vaccin ayant perdu son pouvoir infectant. Cette perte de pouvoir infectant (ou titre) sous l'effet d'une élévation de température, a été clairement mise en évidence dans une étude publiée par l'Organisation Mondiale de la Santé, 1974, 44, 729−737 sous la signature de Y. Robin, A. C. Saenz, A. S. Outschoorn et B. Grab, intitulée »Etude de la thermostabilité du vaccin antiamaril sur des échantillons de huit lots provenant de divers pays« d'où il ressort que les compositions vaccinantes stabilisées par lyophilisation perdent environ 75% de leur pouvoir infectant initial à la suite d'un stockage de 6 mois à +5°C ou de deux semaines à +22°C, et environ 95% de leur pouvoir infectant initial après une semaine à 37°C. C'est la raison pour laquelle on s'est efforcé d'améliorer la thermostabilité des vaccins vivants lyophilisés, en leur ajoutant des stabilisants. C'est ainsi que dans le brevet français 1 548 489 et le Brevet spécial de Médicament 732IM, la Société Recherche et Industrie Thérapeutiques R. I. T. a proposé d'ajouter à du virus vivant atténué de rubéole, avant ou après lyophilisation, un agent stabilisant constitué par de la solution de Hanks additionnée d'hydrolysat de caséine (éventuellement complété par une deuxième solution aqueuse stabilisante contenant du glutamate de potassium, du saccharose et du chloramphénicol). Des résultats relatifs à la thermostabilité de vaccins contre la rougeole lyophilisés et reconstitués ont, par ailleurs, été exposés par cette Société au 15ème Congrès de l'AISB sur les vaccinations dans les pays en voie de développement, qui a eu lieu à la Guadeloupe en 1978, et publiés dans Develop. biol. Standard. vol. 41, p. 259−264 (S. Karger Ed., Bâle, 1978): les expérimentations dont il est rendu compte dans cette publication montrent qu'avec l'agent stabilisant préconisé par la Société R. I. T., la perte de titre est de 0,30 $Log_{10}$ au bout de 2 jours et de 0,70 $Log_{10}$ au bout d'une semaine, pour un vaccin lyophilisé exposé à une température de 41°C au bain-marie pendant 14 jours, puis reconstitué avec du diluant pré-chauffé à 41°C, sans que la reconstitution provoque une diminution supplémentaire du titre. A noter que la solution de Hanks préconisée selon les Brevets R. I. T. est une solution aqueuse contenant des sels minéraux, du glucose et du rouge de phénol et que l'hydrolysat de caséine est essentiellement constitué des acides aminés suivants: acide glutamique, leucine, proline, lysine, valine, tyrosine, méthionine, phénylalanine, arginine, histidine. Il a également été proposé (cf. Brevet français 2 371 927 au nom de Connaught Laboratories Ltd.) de stabiliser un vaccin contre la polyomyélite, par une composition aqueuse stabilisante contenant 0,3−1,0 M environ de tampon Tris (hydroxy-)aminométhane, environ 100 µg/ml de L-cystine et environ, 0,8 mg/ml de vaccin, de gélatine hydrolysée par un acide, la gélatine hydrolysée étant essentiellement constituée des acides aminés suivants: glycocolle, alanine, leucine, phénylalamine, tyrosine, sérine, proline, hydroxyproline, acide aspartique, acide glutamique, arginine, lysine, tryptophane. Le Brevet français Merck & Co. 2 424 031 décrit, quant à lui, un »stabilisateur de vaccin« préconisé pour stabiliser une composition vaccinante à l'état lyophilisé ou liquide (telle que virus de la rougeole, des oreillons, de la rubéole, de la varicelle, de la polyomyélite, de l'hépatite, de l'herpés simplex 1 ou 2, ou de combinaisons de ces virus) à l'aide d'une solution aqueuse stabilisante contenant de la gélatine hydrolysée, un polyalcool tel que le sorbitol et une quantité de tampon acide suffisante pour maintenir le pH entre 6,0 et 6,5 environ, le tampon pouvant être du tampon

0 065 905

phosphate, acétate ou citrate. Dans le »Journal of Biological Standardization« (1980), 8, p. 281—287, W. J. McAleer, H. Z. Markus, A. A. McLean, E. B. Buynak et M. R. Hilleman ont rendu compte des expérimentations qu'ils ont effectuées pour déterminer la stabilité au stockage à différentes températures, de vaccins vivants contre la rougeole, les oreillons et la rubéole, mis en suspension dans ce nouvel agent stabilisant: ils ont montré que le vaccin lyophilisé anti-rougeole perd moins d'un tiers de son pouvoir infectant lorsqu'il a été stocké pendant deux mois à 36—38° ou pendant un mois à 44—46°C.

La présente invention a pour but de pourvoir à un agent de stabilisation de vaccins contenant des virus atténués vivants, qui répond mieux aux nécessités de la pratique que les agents stabilisants proposés conformément à l'Art antérieur, en ce qu'il permet d'obtenir des vaccins vivants stabilisés dont la thermostabilité est notablement améliorée, même lorsque ces vaccins sont soumis à des températures élevées pendant des durées prolongées, par rapport à celle des vaccins stabilisés par les agents stabilisants proposés dans l'Art antérieur, en ce que les agents stabilisants proposés sont de composition plus simple que ceux proposés dans l'Art antérieur et, par conséquent, d'un prix de revient inférieur par rapport à ces derniers, permettant ainsi la production de vaccins stabilisés dans des conditions économiques plus favorables que par le passé et en ce que les agents stabilisants proposés conformément à la présente invention conviennent à l'usage en association avec certains vaccins viraux dont les composants actifs sont thermosensibles, vaccins susceptibles d'être lyophilisés, comme c'est le cas pour le vaccin antiamaril.

La présente invention a pour objet un agent stabilisant de vaccins contenant des virus atténués vivants, caractérisé en ce qu'il est constitué par une solution tampon phosphate (PBS) contenant des ions calcium et magnésium, laquelle contient en outre du lactose, du sorbitol et au moins un du acide aminé naturel.

Comme on le sait, la composition de la solution tampon phosphate PBS contenant des ions calcium et magnésium, qui est bien connue, est la suivante:

| | |
|---|---|
| NaCl | 8 g p. 1000 |
| KCl | 0,2 g p. 1000 |
| $Na_2HPO_4, 2H_2O$ | 1,13 g p. 1000 |
| $K\,H_2PO_4$ | 0,2 g p. 1000 |
| $CaCl_2$ | 0,1 g p. 1000 |
| $MgSO_4, 7H_2O$ | 0,076 g p. 1000 |
| $H_2O$ q. s. p. | 1000 ml |

Conformément à l'invention, la quantité de lactose ajoutée à la solution tampon PBS contenant des ions calcium et magnésium est telle que sa concentration finale dans l'agent stabilisant conforme à l'invention, soit de l'ordre de 4%; la quantité de sorbitol ajoutée à la solution PBS + $Ca^{++}$ et $Mg^{++}$ est telle que sa concentration finale dans l'agent stabilisant conforme à l'invention, soit de l'ordre de 2% et la quantité d'acide(s) aminé(s) ajoutée à la solution PBS + $Ca^{++}$ et $Mg^{++}$ est telle que la concentration finale en acide(s) aminé(s) dans l'agent stabilisant conforme à l'invention soit de l'ordre de 0,005 M à 0,05 M.

L'acide aminé ou les acides aminés mis en oeuvre dans l'agent stabilisant conforme à l'invention, sont choisis de préférence parmi les acides aminés solubles dans les conditions de préparation des vaccins, et notamment, dans le groupe qui comprend l'histidine, l'alanine, la valine, la thréonine, l'arginine, la méthionine, l'hydroxyproline, la lysine, l'isoleucine, la phénylalanine, la sérine.

Selon un mode de réalisation préféré de l'agent stabilisant conforme à l'invention, celui-ci contient de l'histidine et de l'alanine, chacune à une concentration finale de 0,01 M.

La présente invention a également pour objet des vaccins stabilisés contenant des virus vivants atténués, caractérisés en ce que le vaccin est essentiellement constitué par une suspension de virus atténués vivants dans l'agent stabilisant conforme à l'invention, dont la composition a été définie plus haut, puis lyophilisée.

La présente invention a enfin pour objet un procédé de préparation de vaccins stabilisés contenant des virus vivants atténués, caractérisé en ce qu'une préparation concentrée de vaccin vivant atténué, est mise en suspension dans une quantité appropriée d'une solution d'agent stabilisant, telle que définie plus haut, après quoi la suspension ainsi obtenue est lyophilisée, les pastilles de vaccin lyophilisé sont réhydratées — ou reconstituées — au moment de l'emploi, par addition d'une quantité appropriée d'eau distillée.

L'on obtient ainsi des vaccins vivants atténués dont la thermostabilité est particulièrement remarquable et dont la perte de pouvoir infectant est extrêmement faible même lorsqu'ils sont soumis à des températures de l'ordre de 40°C, ce qui permet d'envisager l'utilisation de ces vaccins stabilisés dans des climats tempérés ou tropicaux même en l'absence accidentelle ou temporaire de moyens de réfrigération, avec une excellente sécurité quant à la protection qu'ils sont susceptibles de conférer aux populations auxquelles ils sont administrés.

Les vaccins vivants atténués stabilisés conformément à la présente invention, sont essentiellement les vaccins contre la fièvre jaune et les vaccins contre la rougeole, la rubéole, les oreillons, la grippe, la varicelle.

3

L'invention sera décrite de façon plus détaillée dans le complément de description qui va suivre, en référant à un exemple de préparation d'un vaccin lyophilisé stabilisé conformément à l'invention.

Il doit être bien entendu, toutefois, que cet exemple de préparation est donné uniquement à titre d'illustration de l'objet de l'invention, dont il ne constitue en aucune manière une limitation.

En outre, la thermostabilité des vaccins stabilisés conformément à la présente invention, a été testée sur des vaccins contre la fièvre jaune, souche 17 D, qui sont des vaccins particulièrement fragiles, qui sont très rapidement détruits par la chaleur à des températures aussi basses que +4°C et même plus basses. Leur thermostabilité a été, en outre, comparée d'une part à celle de vaccins antiamarils lyophilisés actuellement fabriqués par Institut Pasteur Production (IPP) et d'autre part à celle d'un vaccin antiamaril stabilisé préparé par la Société Wellcome, qui n'a pas divulgué la composition de l'agent stabilisant qu'elle produit, mais a cependant décrit les résultats qu'elle a obtenus dans »Developp. Biol. Standard.«, vol. 36, p. 279–283 (S. Karger Editeur, Bâle, 1977).

I— Exemple de préparation d'un vaccin antiamaril stabilisé conformément à la présente invention

### 1°— Préparation des virus

On utilise des oeufs de poule embryonnés que l'on incube jusqu'à ce que l'embryon se développe, c'est-à-dire pendant environ 10 jours.

On inocule alors dans la cavité allantoïdienne, la quantité voulue de germes de la souche 17 D du virus de la fièvre jaune; ce germe diffuse et infecte l'oeuf, y compris l'embryon.

Au bout de 4 jours d'incubation, on récolte les embryons, on leur coupe les pattes et la tête, on les broie dans un broyeur à hélice et on recueille le broyat.

### 2°— Préparation de la solution d'agent stabilisant

La solution d'agent stabilisant est préparée en ajoutant à une solution tampon PBS contenant des ions $Ca^{++}$ et $Mg^{++}$, du lactose, du sorbitol et un ou plusieurs acides aminés, pour obtenir la composition suivante:

— Solution tampon PBS contenant des ions $Ca^{++}$ et $Mg^{++}$:

| | |
|---|---|
| NaCl | 8 g p. 1000 |
| KCl | 0,2 g p. 1000 |
| $Na_2HPO_4, 2H_2O$ | 1,13 g p. 1000 |
| $KH_2PO_4$ | 0,2 g p. 1000 |
| $CaCl_2$ | 0,1 g p. 1000 |
| $MgSO_4, 7H_2O$ | 0,07 g p. 1000 |
| $H_2O$ distillée q. s. p. | 1000 ml |

— Lactose pour obtenir une concentration finale de 4%
— Sorbitol pour obtenir une concentration finale de 2%
— Acides aminés: concentration finale 0,02 M

### 3°— Préparation du vaccin stabilisé lyophilisé

Le broyat obtenu en 1°— est mis en suspension dans la solution d'agent stabilisant telle que décrie en 2°— pour obtenir une concentration de 100 g d'embryons/100 ml de solution d'agent stabilisant. Cette suspension concentrée est congelée à —60°C et est conservée à cette température.

### 4°— Préparation d'ampoules injectables du vaccin stabilisé

La suspension concentrée congelée est décongelée, diluée par la solution d'agent stabilisant telle que décrite en 2° ci-dessus, le taux de dilution étant ajusté en fonction du titre en virus de la suspension concentrée. On obtient, après cette dilution, le vaccin final qui est réparti en ampoules, puis lyophilisé.

Au moment de l'emploi, chaque ampoule de vaccin lyophilisé reçoit un volume d'eau distillée égal au volume de vaccin liquide antérieurement introduit dans cette ampoule: le vaccin est ainsi »reconstitué« et est prêt à être injecté.

### II— Essais de stabilité

La Demanderesse a pu établir que l'introduction de sorbitol dans l'agent stabilisant conforme à l'invention, a pour effect de protéger l'infectivité du virus pendant la lyophilisation et que le lactose améliore la cohésion physique de la préparation de vaccin et élève le point de décongélation commençante de la suspension de virus dans l'agent stabilisant.

La Demanderesse a également pu établir que l'effet protecteur exercé par la solution stabilisante sur la préparation virale est essentiellement dû à la présence des acides aminés et aux ions $Ca^{++}$ et $Mg^{++}$ présents dans la solution PBS.

Elle a, en outre, pu mettre en évidence que le mélange stabilisant associé aux virus dans le produit lyophilisé, facilite la réhydratation — ou reconstitution — de ce dernier, en vue de son injection: en effet, alors que la réhydratation du produit lyophilisé classique fabriqué actuellement, en vue de son injection, est difficile à obtenir du fait de la présence d'agrégats de substances organiques dans la pastille lyophilisée, ce n'est pas le cas du produit lyophilisé stabilisé conforme à l'invention, dont la réhydratation est trés facile à obtenir attendu qu'il ne contient pas d'agrégats de substances organiques.

La thermostabilité d'une solution injectable de vaccin stabilisé a été évaluée avec des agents stabilisants conformes à l'invention contenant différents acides aminés et exprimée par la perte de titre, $\overline{m} \pm \frac{ts}{\sqrt{n}}$, en log UFP/ml, du vaccin après exposition de ce dernier pendant 14 jours à 37°C.

Dans l'équation ci-dessus:

$\overline{m}$ = moyenne des valeurs observées pour la perte du titre
t = indice du test de Student
S = écart-type
n = nombre de titrages

et UFP/ml = unité formant plage/ml.

Le Tableau 1 ci-après montre la perte de titre obtenue dans ces conditions, avec une solution d'agent stabilisant contenant un seul des 11 acides aminés testés, à une concentration de 0,01 M, le chiffre du milieu indiquant la valeur moyenne de perte de titre et les deux chiffres qui l'entourent indiquant l'intervalle de confiance des moyennes. Les onze acides aminés testés ont été choisis pour leur bonne solubilité dans la solution tampon PBS contenant des ions $Ca^{++}$ et $Mg^{++}$ dans les conditions de préparation du vaccin.

Tableau 1

Evaluation de la perte de titre d'un vaccin stabilisé par une solution d'agent stabilisant contenant un seul acide aminé à une concentration 0,01 M

| Acide aminé | Perte de titre $\overline{m} \pm \frac{ts}{\sqrt{n}}$ log UFP/ml | | |
|---|---|---|---|
| Histidine (n = 6)*) | 0,343 | 0,438 | 0,532 |
| Alanine (n = 7)*) | 0,318 | 0,450 | 0,581 |
| Valine (n = 6)*) | 0,364 | 0,459 | 0,555 |
| Thréonine (n = 7)*) | 0,378 | 0,466 | 0,553 |
| Arginine (n = 6)*) | 0,340 | 0,492 | 0,643 |
| Méthionine (n = 6)*) | 0,403 | 0,502 | 0,602 |
| Hydroxyproline (n = 7)*) | 0,458 | 0,551 | 0,643 |

5

Fortsetzung

| Acide aminé | | Perte de titre $\overline{m} \pm \dfrac{ts}{\sqrt{n}}$ log UFP/ml |
|---|---|---|
| Lysine | (n = 7)*) | 0,521   0,583   0,644 |
| Isoleucine | (n = 5)*) | 0,323   0,591   0,858 |
| Phénylalanine | (n = 6)*) | 0,435   0,608   0,780 |
| Sérine | (n = 6)*) | 0,600   0,716   0,831 |

*) n = nombre de titrages.

Il ressort de ce Tableau que la perte de titre obtenue aprés exposition d'une solution de vaccin stabilisé conformément à l'invention pendant 14 jours à 37°C, a été très faible pour tous les acides aminés testés et n'a pas excédé, dans le pire des cas, celui de l'isoleucine, 0,858. Ce Tableau fait également apparaître que les pertes de titre les plus faibles ont été obtenues respectivement avec l'histidine et avec l'alanine.

Les expérimentations dont il sera rendu compte ci-après, ont, de ce fait, été poursuivies avec une solution d'agent stabilisant contenant soit de l'histidine seule à une concentration de 0,01 M, soit de l'alanine seule à une concentration de 0,01 M, soit une association d'histidine et d'alanine chacune à une concentration de 0,01 M.

Le Tableau 2 ci-après montre les pertes de titre exprimées en log UFP/ml obtenues respectivement avec des vaccins lyophilisés tels qu'actuellement disponibles dans le commerce et avec des vaccins stabilisés conformément à la présente invention, exposés pendant 14 jours à une température de 37°C.

Les produits (1) et (2) ont été préparés en diluant avec de l'eau distillée, et dans des proportions convenables, la suspension virale concentrée, de façon à obtenir le vaccin final prêt à être lyophilisé.

Pour les produits (3), (4) et (5), la suspension virale concentrée, appartenant à un même lot de préparation, a été diluée dans la solution d'agent stabilisant dont la composition est indiquée dans le Tableau 2, avant de procéder à la lyophilisation du produit final.

Tableau 2

Evaluation comparée de la perte de titre de vaccins du commerce stabilisés par lyophilisation et de vaccins stabilisés conformément à l'invention

| $\overline{m} \pm \dfrac{ts}{\sqrt{n}}$ ($\alpha = 0,05$) log UFP/ml | 0 | 0.5 | 1.0 | 1.5 | 2.0 |
|---|---|---|---|---|---|
| (1) Vaccin en suspension dans eau distillée lyophilisé (lot I. P. P. 130c) (n = 5) | | | | | 1,71 1,83 1,95 |
| (2) Vaccin expérimental préparé en eau distillée (lot 192) (n = 3) | | | | 1,21 1,34 1,48 | |
| (3) Vaccin stabilisé par l'agent stabilisant conforme à l'invention contenant histidine + alanine à une concentration de 0,05 M (n = 12) | | 0,67 0,65 0,73 | | | |

(Suite)

| $\overline{m} \pm \dfrac{ts}{\sqrt{n}}$ ($\alpha = 0{,}05$) log UFP/ml | 0 | 0.5 | 1.0 | 1.5 | 2.0 |

(4) Vaccin stabilisé par l'agent stabilisant conforme à l'invention contenant histidine + alanine à concentration 0,01 M (n = 23)

0,48 0,52 0,56

(5) Vaccin stabilisé par l'agent stabilisant conforme à l'invention contenant histidine + alanine à concentration 0,01 M (n = 23)

0,41 0,46 0,51

Il ressort de ce Tableau que la perte de titre d'un vaccin stabilisé conformément à l'invention à l'aide d'une solution d'agent stabilisant contenant une association d'histidine et d'alanine chacune à une concentration de 0,01 M, est en moyenne 3 à 4 fois inférieure (selon le lot considéré) à celle d'un vaccin préparé en eau distillée et lyophilisée.

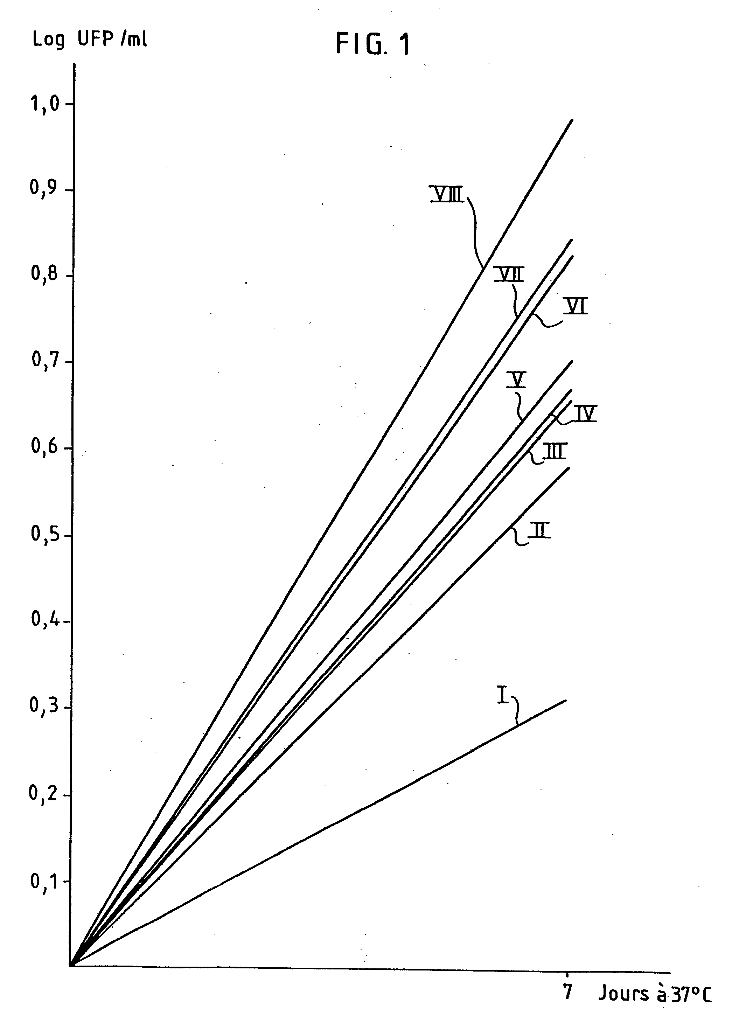

La figure 1 annexée montre les courbes de perte de titre moyenne, exprimée en Log UFP/ml, obtenues après avoir exposé pendant 7 jours à 37°C, des vaccins antiamarils stabilisés par différents moyens, à savoir:

— la courbe I montre la perte de titre d'un vaccin lyophilisé stabilisé par une agent stabilisant conforme à l'invention, contenant du lactose, du sorbitol et, comme acides aminés: de la lysine et de l'hydroxyproline,
— la courbe II montre la perte de titre d'un vaccin lyophilisé stabilisé par du sorbitol auquel a été ajoutée de l'albumine humaine,
— la courbe III montre la perte de titre d'un vaccin lyophilisé stabilisé par du saccharose,
— la courbe IV montre la perte de titre d'un vaccin lyophilisé stabilisé par du lactose,
— la courbe V montre la perte de titre d'un vaccin lyophilisé stabilié par du saccharose auquel a été ajoutée de l'albumine humaine,
— la courbe VI montre la perte de titre d'un vaccin lyophilisé stabilisé par un mélange de lactose et de glutamate de sodium,
— la courbe VII montre la perte de titre d'un vaccin lyophilisé stabilisé par du lactose additionné d'albumine humaine,
— la courbe VIII montre la perte de titre d'un vaccin stabilisé par lyophilisation d'une suspension de virus dans de l'eau distillée.

La figure 1 fait apparaître clairement la remarquable thermostabilité du vaccin stabilisé conformément à la présente invention.

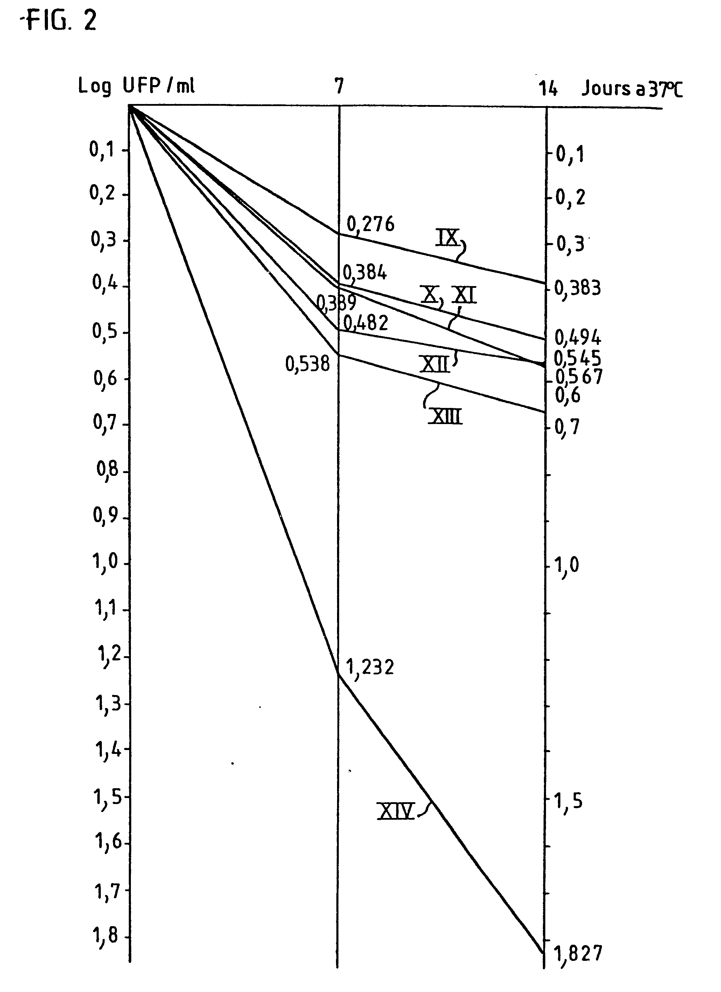

La figure 2 également annexée montre les pertes de titre moyennes, exprimées en Log UFP/ml, relevées pendant 14 jours, subies par des vaccins antiamarils lyophilisés, stabilisés par différents agents stabilisants, ainsi que par un vaccin antiamaril stabilisé par simple lyophilisation, exposés à une température de 37°C, et notamment:

— la courbe IX montre la perte de titre subie par un vaccin lyophilisé stabilisé conformément à l'invention par un agent stabilisant constitué par du PBS + Ca, Mg contenant du lactose, du sorbitol et un mélange de deux acides aminés à une concentration de 0,01 M,
— la courbe X montre le perte de titre subie par un vaccin lyophilisé stabilisé par un agent stabilisant constitué par du PBS dépourvu d'ions $Ca^{++}$ et $Mg^{++}$, mais contenant du lactose, du sorbitol et deux acides aminés à une concentration de 0,01 M,
— la courbe XI montre la perte de titre subie par un vaccin lyophilisé stabilisé uniquement par un mélange de deux acides aminés à une concentration de 0,01 M,
— la courbe XII montre la perte de titre subie par le vaccin Wellcome mentionné dans la publication précitée,
— la courbe XIII montre la perte de titre subie par un vaccin lyophilisé stabilisé par un agent stabilisant constitué par du PBS dépourvu d'ions $Ca^{++}$ et $Mg^{++}$, mais contenant du lactose, du sorbitol et deux acides aminés à une concentration de 0,05 M, et
— la courbe XIV montre la perte de titre subie par un vaccin du commerce stabilisé par simple lyophilisation d'une suspension aqueuse du virus amaril.

La comparaison des courbes IX à XIV est indéniablement en faveur de l'amélioration remarquable de la thermostabilité obtenue avec le vaccin stabilisé conformément à la présente invention.

Ces résultats ont été confirmés par une étude comparative de thermostabilité effectuée sur des lots de vaccin anti-amaril 17 D provenant d'une production à l'échelle industrielle dilués respectivement en eau distillée selon la méthode classique, et en agent stabilisant conforme à l'invention; cette étude comparative a montré que le vaccin préparé avec la solution d'agent stabilisant conforme à l'invention, présente une stabilité remarquable de son titre infectieux aussi bien à 37°C qu'à la température du laboratoire, par raport à un témoin conservé à −60°C.

Cette étude comparative a été effectuée dans les conditions suivantes:

Cinq lots de vaccin anti-amaril 17 D ont été préparés et lyophilisés dans une installation de production industrielle de ce vaccin.

Pour chacun de ces lots, une même suspension concentrée de virus 17 D a été divisée en deux parties. L'une a été diluée en eau distillée pour obtenir le vaccin classique non thermostable. L'autre a été diluée avec une solution protectrice d'agent stabilisant telle que définie plus haut. Les deux parties ont ensuite été lyophilisées simultanément, dans la même opération.

Les Tableaux 3 et 4 qui vont suivre résument les résultats d'épreuves de thermostabilité à +37°C et à la température du laboratoire (environ +20°C).

Les titrages effectués ont mesuré le titre infectieux de chaque échantillon, exprimé en log UFP (Unités formant plages). Pour l'épreuve à 37°C, chaque titrage a été pratiqué sur 3 échantillons introduits dans la même série: témoin conservé à −60°C, échantillon chauffé à 37°C pendant 7 jours, échantillon chauffé à 37°C pendant 14 jours. Pour l'épreuve à la température du laboratoire, la même technique a été appliquée, titrant simultanément les échantillons suivants: témoin conservé à −60°C, échantillons maintenus à la température ambiante pendant 2 semaines, 1 mois, 2 mois, 3 mois et 6 mois. Dans les deux cas, les échantillons soumis à une épreuve et en attente du jour du titrage ont été conservés à −60°C.

Les Tableaux 3 et 4 montrent clairement:

1) que le vaccin préparé en stabilisant a régulièrement un titre infectieux plus élevé que son homologue préparé en eau distillée, et cela au niveau du témoin conservé à −60°C, en dehors de toute épreuve thermique;

2) que la perte de titre est considérablement plus faible avec le vaccin stabilisé, quelles que soient la température et la durée de l'épreuve thermique.

Tableau 3

Vaccin anti-amaril
Epreuve de stabilité à la température du laboratoire
(Valeurs en log UFP. Moyennes de 4 tritrages)

| Lot n° | Vaccin préparé en | Perte de titre après exposition à la température du laboratoire pendant | | | | |
|--------|-------------------|-----------|--------|--------|--------|--------|
|        |                   | 2 semaines | 1 mois | 2 mois | 3 mois | 6 mois |
| 18 | Eau distillée | 0,191 | 0,479 | 0,824 | 0,730 | 1,525 |
|    | Stabilisant   | 0,116 | 0,127 | 0,167 | 0,233 | 0,274 |
| 25 | Eau distillée | —     | 0,369 | 0,592 | 0,859 | 1,818 |
|    | Stabilisant   | —     | 0,098 | 0,153 | 0,139 | 0,185 |
| 26 | Eau distillée | 0,285 | 0,375 | 0,579 | 0,708 | 1,348 |
|    | Stabilisant   | 0,125 | 0,208 | 0,263 | 0,336 | 0,504 |
| 27 | Eau distillée | 0,225 | 0,411 | 0,655 | 0,826 | 1,329 |
|    | Stabilisant   | 0,137 | 0,223 | 0,246 | 0,341 | 0,437 |
| 28 | Eau distillée | 0,266 | 0,409 | 0,582 | 0,727 | 1,025 |
|    | Stabilisant   | 0,014 | 0,134 | 0,222 | 0,238 | 0,302 |

Tableau 4

Vaccin anti-amaril
Epreuve de stabilité à 37°C
(titres en log UFP/ml: moyennes de 4 titrages)

| Lot n° | Vaccin préparé en | Témoin conservé à 37°C | Perte de titre après chauffage à 37°C | |
|---|---|---|---|---|
| | | | 7 jours | 14 jours |
| 18 | Eau distillée | 5,541 | 1,037 | 2,412 |
| | Stabilisant | 5,717 | 0,284 | 0,282 |
| 25 | Eau distillée | 5,543 | 0,876 | 1,575 |
| | Stabilisant | 5,875 | 0,140 | 0,148 |
| 26 | Eau distillée | 5,357 | 0,730 | 1,090 |
| | Stabilisant | 5,638 | 0,352 | 0,464 |
| 27 | Eau distillée | 5,417 | 0,679 | 1,076 |
| | Stabilisant | 5,710 | 0,280 | 0,362 |
| 28 | Eau distillée | 5,109 | 0,604 | 0,977 |
| | Stabilisant | 5,426 | 0,237 | 0,292 |

## Revendications

1. Agent stabilisant de vaccins contenant des virus atténués vivants, caractérisé en ce qu'il est constitué par une solution tampon phosphate (PBS) contenant des ions Calcium et Magnésium, laquelle contient, en outre, du lactose, du sorbitol et au moins un $\alpha$-acide aminé naturel.

2. Agent stabilisant de vaccins selon la Revendication 1, caractérisé en ce que la quantité de lactose qu'il contient est telle que sa concentration finale dans l'agent stabilisant soit de l'ordre de 4%.

3. Agent stabilisant de vaccins selon l'une quelconque des Revendications 1 ou 2, caractérisé en ce que la quantité de sorbitol qu'il contient est telle que sa concentration finale dans l'agent stabilisant soit de l'ordre de 2%.

4. Agent stabilisant de vaccins selon l'une quelconque des Revendications 1 à 3, caractérisé en ce que la quantité d'acide(s) aminé(s) qu'il contient est telle que la concentration finale en acide(s) aminé(s) dans l'agent stabilisant soit de 0,005 M à 0,05 M.

5. Agent stabilisant selon la Revendication 4, caractérisé en ce que l'acide aminé ou les acides aminés mis en oeuvre sont choisis, de préférence, parmi les acides aminés solubles dans les conditions de préparation des vaccins, et notamment, dans le groupe qui comprend l'histidine, l'alanine, la valine, la thréonine, l'arginine, la méthionine, l'hydroxyproline, la lysine, l'isoleucine, la phénylalanine, la sérine.

6. Agent stabilisant selon l'une quelconque des Revendications 1 à 5, caractérisé en ce qu'il contient une association d'histidine et d'alanine, chacune à une concentration de 0,01 M.

7. Vaccin stabilisé contenant des virus vivants atténués, caractérisé en ce qu'il est essentiellement constitué par une suspension de virus atténués vivants dans l'agent stabilisant selon l'une quelconque des Revendications 1 à 6, et lyophilisé.

8. Vaccin stabilisé selon la Revendication 7, caractérisé en ce qu'il contient essentiellement des virus atténués vivants de la rougeole, de la rubéole, des oreillons, de la varicelle, de la grippe, de la fièvre jaune.

9. Procédé de préparation de vaccins stabilisés contenant des virus vivants atténués, caractérisé en ce qu'une préparation de vaccin vivant atténué, est mise en suspension dans une quantité appropriée d'une solution d'agent stabilisant selon l'une quelconque des Revendications 1 à 6, après quoi la suspension ainsi obtenue est lyophilisée, les pastilles de vaccin lyophilisé étant réhydratées — ou reconstituées — en vue de l'utilisation du vaccin, par addition d'une quantité d'eau distillé appropriée.

## Patentansprüche

1. Stabilisator für abgeschwächte lebende Viren enthaltende Vaccinen, dadurch gekennzeichnet, daß er aus einer Calcium- und Magnesiumionen enthaltenden Phosphatpufferlösung (PBS) besteht, die außerdem Laktose, Sorbit und mindestens eine natürliche $\alpha$-Aminosäure enthält.

2. Stabilisator für Vaccinen nach Anspruch 1, dadurch gekennzeichnet, daß die enthaltene Laktosemenge derart ist, daß ihre Endkonzentration in dem Stabilisator in der Größenordnung von 4% liegt.

3. Stabilisator für Vaccinen nach einem der Ansprüche 1 oder 2, dadurch gekennzeichnet, daß die enthaltene Sorbitmenge derart ist, daß seine Endkonzentration in dem Stabilisator in der Größenordnung von 2% liegt.

4. Stabilisator für Vaccinen nach einem der Ansprüche 1 bis 3, dadurch gekennzeichnet, daß die Menge der enthaltenen Aminosäure(n) derart ist, daß die Endkonzentration der Aminosäure(n) in dem Stabilisator 0,005 m bis 0,05 m beträgt.

5. Stabilisator nach Anspruch 4, dadurch gekennzeichnet, daß die eingesetzte Aminosäure oder die eingesetzten Aminosäuren vorzugsweise ausgewählt sind aus den unter den Herstellungsbedingungen der Vaccinen löslichen Aminosäuren, und insbesondere aus der Gruppe die Histidin, Alanin, Valin, Threonin, Arginin, Methionin, Hydroxyprolin, Lysin, Isoleucin, Phenylalanin, Serin umfaßt.

6. Stabilisator nach einem der Ansprüche 1 bis 5, dadurch gekennzeichnet, daß er eine Assoziation von Histiden und Alanin, jedes in einer Konzentration von 0,01 m, umfaßt.

7. Stabilisierte abgeschwächte lebende Viren enthaltende Vaccine, dadurch gekennzeichnet, daß sie im wesentlichen aus einer Suspension von abgeschwächten lebenden Viren in dem Stabilisator gemäß einem der Ansprüche 1 bis 6 besteht und lyophilisiert ist.

8. Stabilisierte Vaccine nach Anspruch 7, dadurch gekennzeichnet, daß sie im wesentlichen abgeschwächte lebende Masern-, Röteln-, Mumps-, Windpocken-, Grippe-, Gelbfieberviren enthält.

9. Verfahren zur Herstellung von abgeschwächten lebenden Viren enthaltenden stabilisierten Vaccinen, dadurch gekennzeichnet, daß ein abgeschwächtes lebendes Vaccinepräparat in einer geeigneten Menge einer Lösung des Stabilisators gemäß einem der Ansprüche 1 bis 6 suspendiert wird, worauf die so erhaltene Suspension lyophilisiert wird, wobei die lyophilisierten Vaccine-Pastillen zur Verwendung der Vaccine durch Zusatz einer geeigneten Menge von destilliertem Wasser erneut hydratisiert — oder rekonstituiert — werden.

## Claims

1. A stabilizing agent for vaccines containing live attenuated viruses, characterized in that it consists of a phosphate buffer solution (PBS) containing calcium and magnesium ions and also containing lactose, sorbitol and at least one natural $\alpha$-aminoacid.

2. A stabilizing agent for vaccines, according to Claim 1, characterized in that the quantity of lactose which it contains is such that its final concentration in the stabilizing agent is of the order of 4%.

3. A stabilizing agent for vaccines, according to either one of Claims 1 and 2, characterized in that the quantity of sorbitol which it contains is such that its final concentration in the stabilizing agent is of the order of 2%.

4. A stabilizing agent for vaccines, according to any one of Claims 1 to 3, characterized in that the quantity of aminoacid(s) which it contains is such that the final concentration of aminoacid(s) in the stabilizing agent is 0,005 M to 0,05 M.

5. A stabilizing agent according to Claim 4, characterized in that the aminoacid or aminoacids used are preferably chosen from aminoacids which are soluble under the conditions of preparation of the vaccines, and especially from the group comprising histidine, alanine, valine, threonine, arginine, methionine, hydroxyproline, lysine, isoleucine, phenylalanine and serine.

6. A stabilizing agent according to any one of Claims 1 to 5, characterized in that it contains a combination of histidine and alanine, each at a concentration of 0,01 M.

7. A stabilized vaccine containing live attenuated viruses, characterized in that it essentially consists of a suspension of live attenuated viruses in the stabilizing agent according to any one of Claims 1 to 6, which is lyophilized.

8. A stabilized vaccine according to Claim 7, characterized in that it essentially contains live attenuated viruses of measles, german measles, mumps, chicken pox, influenza or yellow fever.

9. A process for the preparation of stabilized vaccines containing live attenuated viruses, characterized in that a preparation of live attenuated vaccine is suspended in an appropriate quantity of a solution of stabilizing agent according to any one of Claims 1 to 6, after which the resulting suspension is lyophilized, the pellets of lyophilized vaccine being rehydrated — or reconstituted — for the purpose of using the vaccine, by the addition of an appropriate quantity of distilled water.

Log UFP /ml

FIG. 1

# FIG. 2